# EUROPÄISCHE PATENTANMELDUNG

(11) **EP 2 676 632 A1**
(43) Veröffentlichungstag der Anmeldung: **25.12.2013**
(21) Anmeldenummer: 13405073.1
(22) Anmeldetag: 18.06.2013
(51) Int. Cl.: A61C 8/00

(54) **Keramikimplantat**

(30) Priorität: 20.06.2012 CH 868122012
(71) Anmelder: Z-Systems Schweiz AG, 4702 Oensingen (CH)
(72) Erfinder: Di Girolamo, Rubino, 6315 Oberägeri (CH); Hug, Thomas, 8703 Erlenbach (CH)
(74) Vertreter: Frei Patent Attorneys

(57) **Zusammenfassung**

Es wird ein einstückiges keramisches Zahnimplantat bereitgestellt, mit einem distalen enossalen Gewindebereich (A), einem gingvialen Zwischenbereich (B) und einem proximalen Bereich (C). Im Gewindebereich (A) ist ein Gewinde (1) angeordnet mit einem Kernradius (r_{K}) und einem Aussenradius des Gewindes (r_{A}). Im Zwischenbereich ist eine proximal zugängliche Eindrehgeometrie (5) zum Eindrehen des Gewindebereichs (A) in ein Knochengewebe angeordnet, wobei die Eindrehgeometrie (5) als nicht rotationssymmetrische Aussenstruktur ausgebildet ist. Der proximale Bereich (C) enthält mindestens eine Struktur zur Befestigung eines ein- oder mehrteiligen Aufbauelementes (15). Im Zwischenbereich (B) ist ein Implantatdurchmesser in jeder Schnittfläche senkrecht zur Implantatachse mindestens gleich gross, wie der doppelte Kernradius (r_{K}) des Gewindes(1). Somit ist auch jeder Implantatdurchmesser durch die Eindrehgeometrie (5) mindestens gleich gross wie der doppelte Kernradius (r_{K}) des Gewindes (1).

## Beschreibung

Die Erfindung liegt auf dem Gebiet der Medizinaltechnik und betrifft ein Zahnimplantat, insbesondere ein einstückiges Keramikimplantat, ein Keramikimplantatsystem sowie ein Zahnimplantatsystem und ein Set mit einem Keramikimplantat.

Keramikimplantate und insbesondere Keramikimplantate bestehend aus Zirkonoxidbasierter Keramik weisen gegenüber den bekannten Titanimplantaten verschiedene Vorteile auf. Sie sind von hervorragender Biokompatibilität, da Keramik und insbesondere Zirkonoxid basierte Keramik kaum physiologische Reaktionen und insbesondere keine allergische Reaktionen auslöst. Metallfreie Keramikimplantate sind deshalb für Allergiker sehr geeignet. Darüber hinaus bewirkt die optimale Gewebeverträglichkeit von Keramikimplantaten, eine sehr schnelle Zahnfleischanlagerung an frisch implantierte Zahnimplantate. So entsteht auch dank der weissen Farbe der Zirkonoxid-Keramik eine verbesserte Ästhetik gegenüber den traditionellen Titanimplantaten. Das schöne Einwachsen in das Zahnfleisch bleibt auch längerfristig erhalten, da Keramikimplantate weniger Plaque anlagern und deshalb im Vergleich zu Titanimplantaten Zahnfleischentzündungen und Zahnfleischrückzugserkrankungen seltener sind:

Keramikimplantate haben gegenüber Titanimplantaten jedoch den Nachteil, dass Keramikmaterial, insbesondere Oxidkeramik wie Zirkonoxid-basierte Keramik oder Aluminiumoxid-basierte Keramik ein sprödhartes Material ist. Eine Herausforderung bleibt deshalb die vergleichsweise hohe Bruchanfälligkeit des Keramikmaterials zu überwinden, selbst wenn beispielsweise Yttrium-stabilisierte Zirkonoxid-basierte Keramik bereits eine verbesserte Bruchstabilität besitzt. Die technische Ausgestaltung von Keramikimplantaten muss also auf die sprödharten Materialeigenschaften abgestimmt werden. Ein einteiliges Implantatsystem ist weniger bruchanfällig als ein zweiteiliges Implantatsystem.

Viele Zahnimplantate, Keramikimplantate und andere Implantate, werden in den Kieferknochen geschraubt. Alle in den Knochen geschraubten Implantate, ob einteilig oder zweiteilig, werden in den Knochen geschraubt, indem ein Eindrehwerkzeug an eine Eindrehgeometrie des Implantates angesetzt wird und ein Eindrehmoment auf das Implantat übertragen wird. Falls diese Krafteinwirkung die vergleichsweise geringe Bruchstabilität des Keramikimplantates überdehnt, wird das Implantat beschädigt. Insbesondere bei einer Innenverbindung in Keramik besteht die Gefahr von Brüchen bei Eindrehkräften, welche im oberen Bereich eines üblicherweise angesetzten Drehmomentes von ca. 35 bis 70 Ncm liegen. Implantatbrüche, bei welchen sich auch Keramikstücke vom Implantat lösen können erhöhen das zahnchirurgische Komplikationsrisiko und den Verschleiss von Keramikimplantaten.

Es ist eine Aufgabe der vorliegenden Erfindung, diese Nachteile im Stand der Technik zu überwinden und ein Keramikimplantat bereitzustellen, welches eine verbesserte Bruchstabilität aufweist selbst und auf welches deshalb eine grösseres Eindrehmoment angesetzt werden kann.

Diese Aufgabe wird gelöst mit einem einstückigen keramischen Zahnimplantat mit einem distalen enossalen Gewindebereich, einem gingvialen Zwischenbereich und einem proximalen Bereich. Im Gewindebereich ist ein Gewinde angeordnet mit einem Kernradius und einem Aussenradius des Gewindes. Im Zwischenbereich ist eine proximal zugängliche Eindrehgeometrie zum Eindrehen des Gewindebereichs in ein Knochengewebe angeordnet, wobei die Eindrehgeometrie als nicht rotationssymmetrische Aussenstruktur ausgebildet ist. Der proximale Bereich enthält mindestens eine Struktur zur Befestigung eines ein- oder mehrteiligen Aufbauelementes. Im Zwischenbereich ist ein Implantatdurchmesser in jeder Schnittfläche senkrecht zur Implantatachse mindestens gleich gross, wie der doppelte Kernradius des Gewindes. Somit ist auch jeder Implantatdurchmesser durch die Eindrehgeometrie mindestens gleich gross wie der doppelte Kernradius des Gewindes.

Im vorliegenden Text wird mit Keramikimplantat ein einstückiges keramisches Zahnimplantat bezeichnet, welches aus Keramikmaterial besteht, insbesondere aus Oxidkeramik wie Zirkonoxid-basierte Keramik, insbesondere Yttrium-stabilisieirte Zirkonoxid-basierte Keramik oder Aluminiumoxid-basierte Keramik.

Die axiale Positionierung eines Bereiches oder einer Struktur wird in dieser Anmeldung mit den Begriffen distal und proximal bezogen auf die Implantationsrichtung so charakterisiert, dass die distale Richtung der Richtung zum apikalen Ende des Zahnimplantates (oder eines Zahns) entspricht und die proximale Richtung der koronalen Richtung des Zahnimplantates (oder eines Zahns) entspricht.

Der proximale Bereich des Keramikimplantates dient wie ein Abutment eines zweistückigen Implantates insbesondere der Aufnahme und Befestigung eines einteiligen oder mehrteiligen Aufbauelementes. Der proximale Bereich weist deshalb mindestens eine Struktur auf, an und/oder mittels welcher ein Aufbauelement am Keramikimplantat befestigt wird. Beispielhafte Strukturen sind ein konischer oder zylindrischer Pfosten oder ein Kugelanker.

Der Zwischenbereich des Keramikimplantates enthält die Eindrehgeometrie, welche am proximalen Ende des Zwischenbereichs angeordnet ist. Das proximale Ende des Zwischenbereichs, bzw. das distale Ende des proximalen Bereichs entspricht anatomisch im Wesentlichen dem Zahnfleisch-Durchtrittsbereich. Der Zwischenbereich des Keramikimplantats, welcher in manchen Ausführungsformen distal zur Eindrehgeometrie eine Erweiterungszone und/oder eine Abstandszone enthält, ist im Wesentlichen über seine ganze axiale Länge von Zahnfleisch (Gingvia) umgeben. Das Keramikimplantat eignet sich je nach axialer Länge des Zwischenbereichs für verschiedene Gingviahöhen. Die axiale Länge des Zwischenbereichs hat beispielsweise ein Mass zwischen 1.5 und 5.5 mm, zum Beispiel 2.5mm, 3.5 mm oder 4.5 mm.

Der ennossale Gewindebereich des Keramikimplantates ist distal zum Zwischenbereich angeordnet und dient der Verankerung des Keramikimplantates im Knochen. Das proximale Ende des Gewindes oder in manchen Ausführungsformen des proximale Ende des Gewindeauslaufs definiert das proximale Ende des Gewindebereichs und gleichzeitig auch das distale Ende des Zwischenbereich.

Als Implantatradius wird im vorliegenden Text der Abstand zwischen der Implantatachse und der Implantatsoberfläche senkrecht zur Achse bezeichnet. In Bereichen des Implantates mit einem nicht kreisförmigen Querschnitt, insbesondere betreffend die nicht rotationssymmetrische Eindrehgeometrie ist der kleinste Implantatradius definiert als Innenradius und er entspricht dem kleinsten Abstand zwischen der Implantatachse und der Implantatsoberfläche senkrecht zur Achse; und analog ist der grösste Implantatradius definiert als der Aussenradius und er entspricht dem grössten Abstand zwischen der Implantatachse und der Implantatsoberfläche senkrecht zur Achse. Der kleinste Durchmesser einer Schnittfläche senkrecht zur Implantatachse des Implantates ist definiert als die kürzeste Verbindungsstrecke zwischen zwei Punkten auf der Implantatsoberfläche, welche durch die Implantatachse führt. Bei einem regelmässigen geradzahligen Aussenkant, z.B. Aussen-Sechskant, entspricht der kleinste Implantatdurchmesser dem doppelten Innenradius.

Allgemein, wenn nichts anderes vermerkt wird, ist der Implantatdurchmesser im vorliegenden Text definiert als der mittlere Durchmesser einer Schnittfläche senkrecht zur Achse. Der Implantatdurchmesser ist im Falle einer kreisförmigen Schnittfläche der Kreisdurchmesser. Im Falle einer nicht kreisförmigen Schnittfläche senkrecht zur Implantatsachse, d.h. beispielsweise eine Schnittfläche durch die Eindrehgeometrie im Zwischenbereich des Implantates, ist der Implantatdurchmesser der Kreisdurchmesser jenes Kreises mit Mittelpunkt auf der Implantatachse, welcher eine gleich grosse Fläche wie die nicht kreisförmige Schnittfläche durch das Implantat aufweist. Der kleinste oder grösste Implantatdurchmesser in einem axialen Bereich des Implantates, beispielsweise im Zwischenbereich, im Bereich der Eindrehgeometrie oder im Gewindebereich ist also der kleinste oder grösste Wert des mittleren Durchmessers aller Schnittflächen senkrecht zur Implantatachse in diesem Bereich. Der Implantatdurchmesser des Keramikimplantates am proximalen Ende des Gewindes hat beispielsweise eine Länge von 3 mm bis 4.5 mm, insbesondere 3.5 mm bis 4.0 mm.

Der Kernradius des Gewindes ist im vorliegenden Text definiert als der Innenradius des Gewindes am proximalen Ende des Gewindes. Der Kerndurchmesser ist definiert als der doppelte Kernradius am proximalen Ende des Gewindes. In manchen Ausführungsformen ist proximal zum proximalen Ende des Gewindes ein Gewindeauslauf angeordnet. In manchen Ausführungsformen des Gewindeauslaufs vergrössert sich der Kernradius in proximaler Richtung, während der Aussenradius des Gewindes unverändert bleibt. Der Aussenradius des Gewindes und des Gewindeauslaufs ist der grösste Radius des Gewindes am proximalen Ende des Gewindes.

Die Grösse des kleinsten Implantatdurchmessers des Implantates ist ein Faktor, welcher die Bruchfestigkeit des Keramikimplantates wesentlich mitbestimmt. Im Allgemeinen gilt, je grösser der kleinste Implantatdurchmesser des Implantates und insbesondere der kleinste Implantatdurchmesser im Bereich der Eindrehgeometrie ist, umso weniger bruchanfällig ist das Keramikimplantat. Es ist ein grosser Vorteil der Keramikimplantate gemäss der vorliegenden Erfindung, dass der kleinste Durchmesser der Eindrehgeometrie nicht kleiner ist als der Kerndurchmesser des Gewindes und somit die Bruchfestigkeit des erfindungsgemässen Keramikimplantates im Vergleich zu bekannten Keramikimplantaten erhöht wird.

Der Innenradius der nicht rotationssymmetrischen Aussenstruktur der Eindrehgeometrie bezeichnet den kleinsten Abstand zwischen der Implantatachse und der Implantatsoberfläche senkrecht zur Achse. Der Aussenradius der nicht rotationssymmetrischen Aussenstruktur der Eindrehgeometrie bezeichnet den grössten Abstand zwischen der Implantatachse und der Implantatsoberfläche senkrecht zur Achse. In manchen Ausführungsformen des Keramikimplantats weist die nicht rotationssymmetrische Aussenstruktur der Eindrehgeometrie einen Innenradius auf, welcher gleich gross oder grösser ist wie der Kernradius des Gewindes.

In manchen Ausführungsformen des Keramikimplantats ist der Unterschied zwischen dem Aussenradius und dem Innenradius der Aussenstruktur der Eindrehgeometrie klein, insbesondere kleiner als 0.3 mm oder 0.2 mm, und beispielsweise ein Mass in einem Bereich zwischen 0.1 mm oder 0.15 mm als Untergrenze und 0.16 mm oder 1.8 mm als Obergrenze aufweist und insbesondere zwischen 0.13 mm und 0.18, beispielsweise 0.15 mm misst. Eine nahezu runde Form der nicht rotationssymmetrischen Aussenstruktur und insbesondere konvexe Aussenstruktur der Eindrehgeometrie liegen einer anatomischen Form sehr nahe.

In manchen Ausführungsformen des Keramikimplantats ist der Aussenradius der Eindrehgeometrie mindestens gleich gross ist wie der Ausserradius des Gewindes.

In manchen Ausführungsformen enthält weist der Zwischenbereich des Keramikimplantates zusätzlich zur Eindrehgeometrie eine Erweiterungszone und/oder eine Abstandszone auf. Die Erweiterungszone ist distal zur Eindrehgeometrie und proximal zum Gewindebereich angeordnet und grenzt in manchen Ausführungsformen direkt an das proximale Ende des Gewindebereichs, insbesondere unmittelbar an das Gewinde oder den Gewindeauslauf. Der Implantatdurchmesser ist am distalen Ende der Erweiterungszone in manchen Ausführungsformen gleich gross wie und in ändern Ausführungsformen grösser als der doppelte Aussenradius des Gewindes und er erweitert sich in der Erweiterungszone in proximaler Richtung. Am proximalen Ende der Erweiterungszone ist der Implantatdurchmesser grösser als an ihrem distalen Ende. Die Erweiterungszone ist in manchen Ausführungsformen als tulpenförmige Erweiterung ausgebildet. welche in einer S-Kurve auskragt.

Die Erweiterungszone ist geeignet, den Implantatdurchmesser im Zwischenbereich in proximaler Richtung zu vergrössern. Bei manchen Ausführungsformen von Implantaten beispielsweise mit einem vergleichsweise kleinen Implantatdurchmesser im Gewindebereich nimmt der Implantatdurchmesser in der Erweiterungszone in proximaler Richtung zu. Auf diese Weise können Sets von Keramikimplantaten mit unterschiedlichem Gewindedurchmesser bereitgestellt werden, deren Eindrehgeometrien nicht rotationssymmetrischen Aussenstrukturen mit identischen Massen aufweisen wie beispielsweise gleiche Innen- und Aussenradien eines Gleichdick.

In manchen Ausführungsformen ist die Erweiterungszone des Zwischenbereichs zum Gewinde beabstandet, das heisst der Implantatsdurchmesser erweitert sich nicht unmittelbar proximal angrenzend zum Gewinde, sondern der Zwischenbereich weist eine Abstandszone auf. Die Abstandszone ist in Ausführungsformen mit einer Erweiterungszone zwischen dem proximalen Ende des Gewindebereichs und dem distalen Ende der Erweiterungszone oder in Ausführungsformen ohne Erweiterungszone zwischen dem proximalen Ende des Gewindebereichs und dem distalen Ende der Eindrehgeometrie angeordnet. In der Abstandszone ist der Implantatradius mindestens gleich gross oder grösser als der Kernradius des Gewindes und im Wesentlichen gleich gross wie der Aussenradius des Gewindes oder Gewindeauslaufs, insbesondere ist der Implantatradius gleich gross wie der Aussenradius des Gewindes oder Gewindeauslaufs.

In manchen Ausführungsformen des Keramikimplantats mit Erweiterungszone ist der Aussenradius der Eindrehgeometrie höchstens gleich gross wie ein Ausserradius der insbesondere tulpenförmigen Erweiterungszone an deren proximalen Ende.

In manchen Ausführungsformen ist der Implantatdurchmesser der Eindrehgeometrie etwas kleiner als der Implantatdurchmesser am proximalen Ende der Erweiterungszone, beispielsweise weniger als 0.3 mm oder 0.2 mm kleiner und insbesondere zwischen 0.13 mm und 0.18 mm kleiner, jedoch ist der Implantatdurchmesser im ganzen Zwischenbereich, also auch jeder Implantatdurchmesser durch die Eindrehgeometrie gleich gross oder grösser wie der Kerndurchmesser des Gewindes.

In manchen Ausführungsformen misst der Implantatdurchmesser am proximalen Ende der Erweiterungszone mindestens 125%, insbesondere mindestens 150%, 175%, 200% oder 225% des Implantatdurchmessers am proximalen Ende des Gewindebereichs Im Gegensatz zu den vorliegend beschriebenen Keramikimplantaten, ist bei Keramikimplantaten, welche aus dem Stand der Technik bekannt sind, die Eindrehgeometrie im proximalen Bereich angeordnet und nicht im Zwischenbereich. Deshalb weisen diese bekannten Keramikimplantate einen vergleichsweise kleineren Implantatsdurchmesser im Bereich der Eindrehgeometrie sowie eine höhere Bruchanfälligkeit auf.

Ein grosser Vorteil der Anordnung der proximal zugänglichen Eindrehgeometrie im Zwischenbereich, in welchem jeder Implantatdurchmesser mindestens gleich gross ist wie der Kerndurchmesser im Gewindebereich, ist der vergrösserte Abstand der Eindrehgeometrie zur Implantatachse. Eine bestimmte Kraft, welche auf die Eindrehgeometrie eines erfindungsgemässen Keramikimplantat angesetzt wird, bewirkt ein grösseres Drehmoment, als eine gleich grosse Kraft, welche auf ein herkömmliches Implantat, dessen Eindrehgeometrie einen kleineren Abstand zur Implantatachse aufweist, bewirken würde. Durch die Vergrösserung des Abstandes der Eindrehgeometrie zur Implantatachse bewirkt eine vergleichsweise kleinere Kraft ein ausreichend grosses Drehmoment, um das Implantat in den Knochen einzudrehen, und belastet das sprödharte Keramikmaterial des Implantates weniger. Dies senkt die Bruchanfälligkeit des erfindungsgemässen Keramikimplantates.

Die Erniedrigung des Bruchrisikos durch die erfindungsgemässe Anordnung der Eindrehgeometrie im Zwischenbereich mit einer Dimensionierung des Implantatdurchmessers, so dass er mindestens gleich gross ist wie der grösste Gewindedurchmesser, erweist sich deshalb als besonders vorteilhaft bei Keramikimplantaten mit einem selbst-schneidenden Gewinde. Denn auf die Eindrehgeometrie eines Implantats mit selbst-schneidendem Gewinde muss eine erhöhte Kraft angesetzt werden, um das Implantat in das Knochengewebe einzudrehen im Vergleich zum Eindrehen eines Implantats in eine Gewindebohrung, welche in das Knochengewebe vorgebohrt wurde. Deshalb sind insbesondere herkömmliche Keramikimplantate mit selbst-schneidendem Gewinde bruchanfällig.

Ein weiterer grosser Vorteil der Anordnung der Eindrehgeometrie im Zwischenbereich ist dass diese nicht versehrt wird, wenn das Implantat im proximalen Bereich beschliffen wird oder bricht und deshalb immer noch vorhanden ist, um das Implantat aus dem Knochen herauszudrehen, falls es entfernt werden muss.

Ein weiterer Vorteil der Anordnung der Eindrehgeometrie näher zum proximalen Ende des Gewindebereichs entsteht durch eine sehr feine aber sichtbare Linie auf der Implantatsoberfläche an der Grenze zwischen dem Gewinde und der Eindrehgeometrie bzw. zur Erweiterungszone oder Abstandszone. Dies stellt für den Zahnarzt eine Implantationshilfe dar. Dies trifft selbst bei einer sehr anatomischen Ausbildung der nicht rotationssymmetrischen Aussenstruktur zu, welche beispielsweise als nahezu rundes konkaves Fünf-Gleichdick ausgebildet ist, und welche beispielsweise nur im Bereich von weniger als 3 mm, insbesondere weniger als 2 mm zurückversetzt ist oder hervorspringt.

Ein weiterer Vorteil der Anordnung der proximal zugänglichen Eindrehgeometrie im Zwischenbereich, ist die Verschiebung der Eindrehgeometrie in distale Richtung, so dass zusätzliche Freiheitsgrade für die technische Ausgestaltung, wie die Gestaltung der Form und Oberfläche, des proximalen Bereichs und somit für die Gestaltung der mindestens einen Struktur zur Befestigung eines Aufbauelementes gewonnen werden. Beispielsweise kann die axiale Länge des proximalen Bereichs vergleichsweise kürzer sein als in Keramikimplantaten aus dem Stand der Technik, da die Eindrehgeometrie nicht auch in diesem proximalen Bereich angeordnet ist. Die Wahl der Form und die technische Ausgestaltung des proximalen Bereichs ist im Wesentlichen unabhängig von der weiter distal angeordneten Eindrehgeometrie. Dies vergrössert die Anpassungsmöglichkeiten des proximalen Bereichs an Parameter, welche das Aufbauelement erfordert.

Ein weiterer Vorteil der Anordnung der Eindrehgeometrie im Zwischenbereich ist die Möglichkeit eine einzige Eindrehgeometrie mit gleich bleibender Form und Grösse für eine Anzahl von verschiedenen Keramikimplantaten als Keramikimplantatsystem bereitzustellen. Durch die Anordnung der Eindrehgeometrie im Zwischenbereich ist ihre Ausgestaltung und ihr Implantatdurchmesser unabhängig wählbar von der Ausgestaltung des proximalen Bereichs und dessen mindestens einen Struktur zur Befestigung des Aufbauelementes und auch unabhängig vom Implantatdurchmesser im distalen enossalen Bereich.

Ein weiterer Aspekt der Erfindung betrifft also ein Keramikimplantatsystem mit mindestens zwei Keramikimplantaten, wobei diese Keramikimplantate mit einer gleichen nicht rotationssymmetrischen Aussengeometrie der Eindrehgeometrie ausgerüstet sind. Eine gleiche nicht rotationssymmetrische Aussenstruktur bedeutet, dass die Aussenstruktur funktionell gleich ist, das heisst, dass sie mit einem strukturell identischen Eindrehwerkzeug funktionell so zusammenwirkt, dass mittels Ansetzen des Werkzeug ein Drehmoment auf das Keramikimplantat übertragen werden kann und es beispielsweise in den Knochen eindrehbar ist. In manchen Ausführungsformen der gleichen nicht rotationssymmetrischen Aussengeometrie sind die Schnittflächen der Eindrehgeometrie senkrecht zur Implantatachse deckungsgleich. Manche Ausführungsformen des Keramikimplantatsystems enthalten zusätzlich ein Eindrehwerkzeug, welches mit der funktionell gleichen Aussenstruktur der Eindrehgeometrie zusammenwirkt.

So weisen manche Ausführungsfonnen des Keramikimplantatsystems mindestens zwei Keramikimplantate mit einer gleichen, im Sinne von funktionell gleichen Eindrehgeometrie auf oder insbesondere mit einer identischen Form und Grösse der Eindrehgeometrie auf, bei welchen sich beispielsweise der proximale Bereich dieser Keramikimplantate unterscheidet beispielsweise in Bezug auf die axiale Länge oder Implantatdurchmesser oder indem der proximale Bereich mit Patrizen für verschiedene Matrizensysteme ausgerüstet ist. In weiteren Ausführungsformen des Keramikimplantatsystems unterscheiden sich die Keramikimplantate alternativ oder zusätzlich im Gewindebereich und weisen beispielsweise unterschiedliche axiale Längen oder Implantatdurchmesser des Gewindebereichs auf, insbesondere einen unterschiedlichen Aussendurchmesser des Gewindes an dessen proximalen Ende. In manchen Ausführungsformen weist die Eindrehgeometrie mit einer gleichen Aussengeometrie, im Sinne der oben beschriebenen funktionell gleichen Aussengeometrie, eine unterschiedliche axiale Länge auf. So unterscheiden sich die Keramikimplantate eines Keramikimplantatsystems alternativ oder zusätzlich zu Unterschieden im proximalen und / oder im Gewindebereich also auch im Zwischenbereich, wie oben genannt beispielsweise in der axialen Länge der Eindrehgeometrie oder beispielsweise zusätzlich oder alternativ auch im Vorhandensein oder der Ausgestaltung der optionalen Erweiterungszone und/oder Abstandszone.

Vorteilhafterweise muss für ein Keramikimplantatsystem enthaltend eine Mehrzahl von Keramikimplantaten nur ein einziges Eindrehwerkzeug bereitgestellt werden, welches mit einer solchen funktionell gleichen Eindrehgeometrie insbesondere von identischer Form und Grösse für verschiedene Ausführungsformen des Keramikimplantates bzw. für ein ganzes Keramikimplantatsystem mit einer Mehrzahl von Keramikimplantaten funktionell zusammenwirkt.

Die im Zwischenbereich angeordnete Eindrehgeometrie einteiliger Keramikimplantate ist typischerweise eine Aussenstruktur. Gängige Eindrehgeometrien sind Aussenpolygonprofile, insbesondere ein Aussen-Sechskant oder ein Aussen-Achtkant oder ein Aussen-Zwölfkant. Beim Eindrehen eines Implantates in den Knochen mittels einer Aussengeometrie entstehen im Gegensatz zum Eindrehen mittels Innengeometrie keine Zugkräfte im Implantat. Solche Zugkräfte im Implantat entstehen typischerweise durch das Aufdrücken eines Eindrehinstrumentes in eine Innengeometrie und das sprödharte Keramikmaterial ist auf solche Zugkräfte sehr empfindlich.

Manche Ausführungsformen weisen als Eindrehgeometrien eine konvexe oder konkave mehrlappige (multi-lobe) Aussenstruktur auf, wie beispielsweise ein regelmässiges oder unregelmässiges Gleichdick. Ein Gleichdick (engl. curve of equal width) ist eine geschlossene Kurve konstanter Breite. Dabei ist die Breite definiert als Abstand zwischen zwei parallelen Geraden, welche die geschlossene Kurve auf gegenüberliegenden Seiten berühren. Die triviale Form des Gleichdick, nämlich die Kreisform ist ausgeschlossen, da sie keine Übertragung von Drehmomenten zulässt. Bevorzugt sind Gleichdick-Aussenstrukturen regelmässiger Gleichdick, welche bezüglich einer axialen Drehung um einen Symmetriewinkel symmetrisch sind. Regelmässige Gleichdick beruhen auf regelmässigen Polygonen mit einer ungeraden Anzahl von Ecken. Ein weiterer Vorteil Eindrehgeometrien in der Form eines Gleichdick ist ihre Herstellbarkeit durch abtragende Verfahren.

Die Eindrehgeometrie kann zylindrisch sein, d.h. entlang der Achse translationssymmetrisch oder konisch, d.h. sich in einem Bereich unter Beibehaltung der Form der Umfangslinie in Funktion der axialen Position stetig verkleinernd oder vergrössernd oder sie kann auch beispielsweise in einem Schnitt parallel zur Achse eine leicht konvexe oder leicht konkave Form aufweisen.

Der kleinste Implantatdurchmesser der Eindrehgeometrie des Keramikimplantates ist jedoch in allen Ausführungsformen mindestens gleich gross wie der Kerndurchmesser des Gewindes. In manchen Ausführungsformen des Keramikimplantates ist der Implantatdurchmesser der Eindrehgeometrie mindestens 10%, 15%, 25% oder 35% grösser als der Kerndurchmesser, insbesondere ist er mindestens 12%, 14%, 16%, 18%, 20%, 22%, 24%, 26%, 28%, 30% oder 32% grösser bezogen auf den Gewindedurchmesser. Die Spannungsverteilung im Keramikimplantat ist bei einer als Aussenstruktur ausgebildeten Eindrehgeometrie mit vergleichsweise grossem Implantatsdurchmesser günstiger als bei Innengeometrien, welche mit dünnen Wandstärken des Keramikimplantates einhergehen und deshalb ein Bruchrisiko begünstigen.

Der proximale Bereich ist proximal zum Zwischenbereich und somit auch proximal zur Eindrehgeometrie angeordnet. In manchen Ausführungsformen ist der grösste Implantatdurchmesser im proximalen Bereich höchstens gleich gross oder kleiner als der grösste Implantatdurchmesser der Eindrehgeometrie. In manchen Ausführungsformen der grösste Implantatdurchmesser im proximalen Bereich höchstens gleichgross wie der grösste Implantatdurchmesser Gewindebereich und insbesondere höchstens gleichgross ist wie der grösste Gewindedurchmesser.

Insbesondere weisen manche Ausführungsformen des Keramikimplantats im proximalen Bereich in jeder Schnittfläche senkrecht zur Implantatachse einen

Implantatdurchmesser auf, welcher kleiner ist als der doppelte Innenradius der Eindrehgeometrie. Der grösste Implantatdurchmesser solcher Ausführungsformen des Keramikimplantates ist dann im Zwischenbereich angeordnet.

In manchen Ausführungsformen des Keramikimplantates weist der proximale Bereich eine zylindrische insbesondere eine kreiszylindrische Form auf. In weiteren Ausführungsformen des Keramikimplantates nimmt der Implantatdurchmesser ab der Grenze zum Zwischenbereich in proximaler Richtung kontinuierlich ab. In weiteren Ausführungsformen des Keramikimplantates wechseln sich Zu- und Abnahme des Implantatdurchmesser ab, beispielsweise so, dass der Implantatdurchmesser ab der Grenze zum Zwischenbereich in proximaler Richtung zuerst abnimmt und sich dann wieder erweitert und/oder er bildet beispielsweise in proximaler Richtung zuerst eine Einschnürung und dann ein Köpfchen. Eine derartige Form mit einem Köpfchen im proximalen Bereichs ist besonders geeignet für ein Keramikimplantat mit einem Kugelanker (engl. Lockball) für das Aufbauelement.

In manchen Ausführungsfonnen ist die mindestens eine Struktur zur Befestigung eines ein- oder mehrteiligen Aufbauelementes als Patrize ausgebildet, welche mit einem als Matrize ausgebildeten Aufbauelement verbindbar ist. In manchen Ausführungsformen enthält die mindestens eine Struktur zur Verbindung eines Aufbauelementes eine Patrize, welche mit einem als Matrize ausgebildeten Aufbauelement verbindbar ist. Solche Matrizen und insbesondere auch Matrizensysteme, sind im Stand der Technik bekannt als Verbindungselemente zur Fixierung von einem gegebenenfalls abnehmbarem Zahnersatz auf einer mit der Matrize verbindbaren Patrize. In solchen Ausführungsformen des erfindungsgemässen einteiligen Keramikimplantates ist also die Patrize ein proximaler Bestandteil des Keramikimplantates während bei bekannten zweiteiligen Implantaten üblicherweise eine Patrize in das im Knochen verankerte Implantat eingeschraubt wird. Insbesondere weist in manchen Ausführungsformen des Keramikimplantates der proximale Bereich eine Patrize auf, welche kompatibel ist mit einem handelsüblichen Patrizen-Matrizensystem wie beispielsweise eine Locator Patrize für ein Locator Matrizensystem, beispielsweise das Novaloc^{™} Matrizensystem oder eine Lockball Patrize für ein Lockball Matrizensystem, beispielsweise das Pro-Snap Matrizensystem von Cendres Metaux.

Vorteilhafterweise kann auf Ausführungsformen des Keramikimplantates mit einem Kugelanker ein Aufbauelement in jeder beliebigen Drehposition aufgesetzt werden. So kann der Zahnchirurg den Gewindebereichs ohne Rücksicht auf die Drehposition so weit eindrehen bis die axiale Höhe des implantierten Implantates optimal eingestellt ist. Ein Keramikimplantat ausgerüstet mit einem Kugelanker im proximalen Bereich stellt deshalb vergleichsweise weniger hohe Anforderungen an die Implantationsgenauigkeit.

In manchen Ausführungsformen des Keramikimplantates steht der proximale Bereich, bzw. die Implantatachse im proximalen Bereich in einem Winkel von bis zu 25° relativ zur distal angrenzenden Eindrehgeometrie, bzw. zur Implantatachse durch die Eindrehgeometrie. In manchen Ausführungsformen des Keramikimplantates ist beispielsweise ein proximaler Teil als abgewinkelter Pfosten ausgebildet.

Manche Ausführungsformen des Keramikimplantats weisen im proximalen Bereich Strukturen auf wie beispielsweise eine Rippung oder dergleichen zur zusätzlichen Befestigung oder Verdrehsicherung eines der Teile des ein- oder mehrteiligen Aufbauelementes oder Rillen und dergleichen beispielsweise zur Aufnahme einer eines Schnappelementes, beispielsweise eines Schnapprings oder einer Dichtung. Ein weiterer Aspekt der Erfindung betrifft ein Zahnimplantatsystem aufweisend ein Keramikimplantat und zusätzlich aufweisend ein einteiliges Aufbauelement oder mindestens einen Teil eines mehrteiligen Aufbauelementes, insbesondere ein Halteelement. Das Halteelement ist in manchen Ausführungsformen eine Matrize, welche an der oder mittels der mindestens einen Struktur im proximalen Bereich am Implantat befestigbar ist.

Ein mehrteiliges Aufbauelement enthält oder besteht aus beispielsweise einem Halteelement, insbesondere aus einer Matrize, und einem Zahnersatz wie eine Krone oder eine Zahnprothese. In manchen Ausführungsformen des Zahnimplantatsystems enthält es beispielsweise das Keramikimplantat und mindestens eine ein- oder mehrteilige Matrize.

In manchen Ausführungsformen eines solchen mehrteiligen Aufbauelementes ist das Halteelement permanent oder lösbar mit dem proximalen Bereich des Keramikimplantates verbindbar und / oder das Haltelement ist permanent oder lösbar mit dem Zahnersatz verbindbar. In manchen Ausführungsformen des Halteelementes ist es mehrteilig, in andern Ausführungsformen ist es einteilig. In manchen Ausführungsformen ist das Halteelement als ein- oder mehrteilige Matrize für einen Kugelanker ausgebildet.

Ein weiterer Gegenstand der Erfindung ist ein Set mit mindestens einem Keramikimplantat und einem Eindrehwerkzeug. Optional enthält das Set mehr als ein Keramikimplantat, insbesondere ein Keramikimplantatsystem. Das Eindrehwerkzeug wirkt zusammen mit der Eindrehgeometrie des mindestens einen Keramikimplantates. Das Set enthält optional zusätzlich ein ein- oder mehrteiliges Aufbauelement wie beispielsweise Halteelemente, andere Prothetikteile, oder Trägerelemente für einen Zahnersatz wie Prothesen, Brücken, Kronen und dergleichen. Das Set enthält mindestens ein Aufbauelement oder mindestens einen Teil des Aufbauelementes welches oder welcher für die unmittelbare Befestigung am Keramikimplantat vorgesehen ist.

Das Keramikimplantat, das Keramikimplantatsystem oder das Zahnimplantatsystem und das Set können in einer sterilen Verpackung vorliegen.

Nachfolgend werden Ausführungsbeispiele der Erfindung anhand von Figuren ausführlicher beschrieben. Die Erfindung ist nicht auf die in den Figuren dargestellte Kombination von Merkmalen der Erfindung limitiert. Insbesondere ist es auch möglich Merkmale, welche für einen ersten der drei Bereiche, Gewindebereich, Zwischenbereich, proximaler Bereich des Keramikimplantates, spezifisch offenbart sind, mit allen offenbarten Ausführungsformen der beiden andern Bereiche zu kombinieren und nicht nur mit jener oder jenen Ausführungsformen der andern Bereiche, welche beispielhaft in Kombination mit dem ersten Bereich beschrieben sind oder in den Figuren dargestellt sind. Die Figuren sind nicht zwingend massstäblich. Gleiche Bezugszeichen bezeichnen gleiche oder analoge Elemente.

Auflistung der Figuren:
- **Fig. 1a und b**: zeigen beispielhafte Ausführungsformen Ia und Ib eines Keramikimplantats mit einer Erweiterungszone in einer schematischen Aufsicht.
- **Fig. 2**: zeigt einen Längsschnitt durch ein beispielhaftes Keramikimplantat II.
- **Fig. 3**: zeigt eine schematische Teilaufsicht auf ein Ausführungsbeispiel eines Keramikimplantatsystems enthaltend beispielhafte Keramikimplantate III und IV mit einer gleichen nicht rotationssymmetrischen Aussengeometrie der Eindrehgeometrie und unterschiedlichem Aussenradius des Gewindes.
- **Fig. 4**: zeigt eine schematische Teilaufsicht auf eine Ausführungsform des Keramikimplantatsystems mit drei Ausführungsformen V, VI und VII des Keramikimplantats mit gleicher nicht rotationssymmetrischer Aussenstruktur einer Eindrehgeometrie mit unterschiedlicher axialer Länge.
- **Fig. 5**: zeigt einen Querschnitt durch eine beispielhafte Ausführungsform der Eindrehgeometrie
- **Fig. 6**: zeigt eine beispielhafte Ausführungsform eines Aufbauelementes für ein Keramikimplantat mit einem Kugelanker

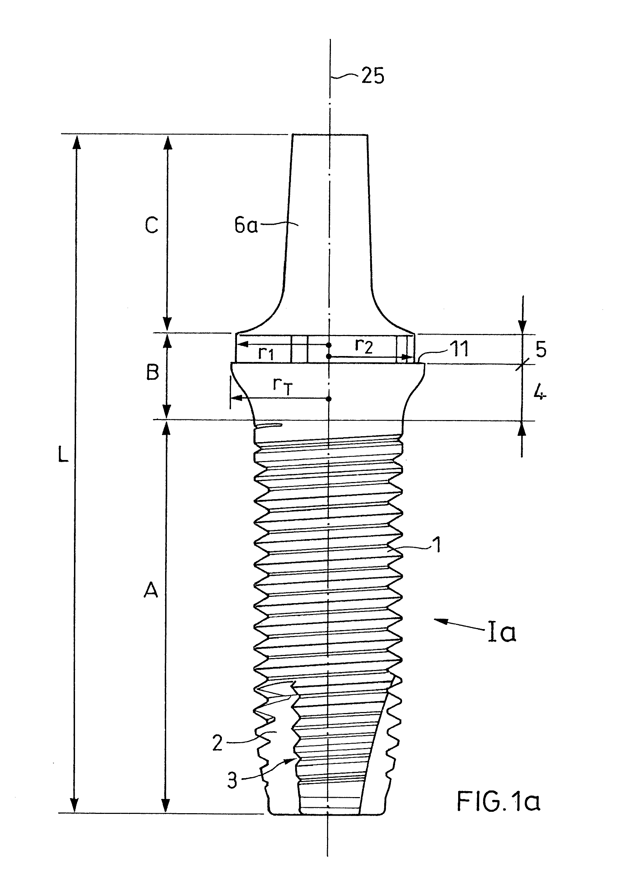

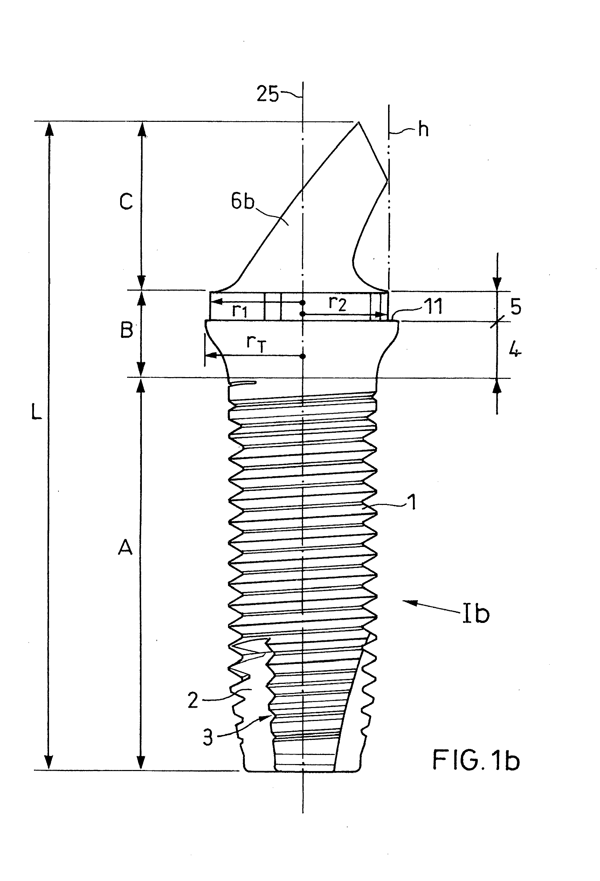

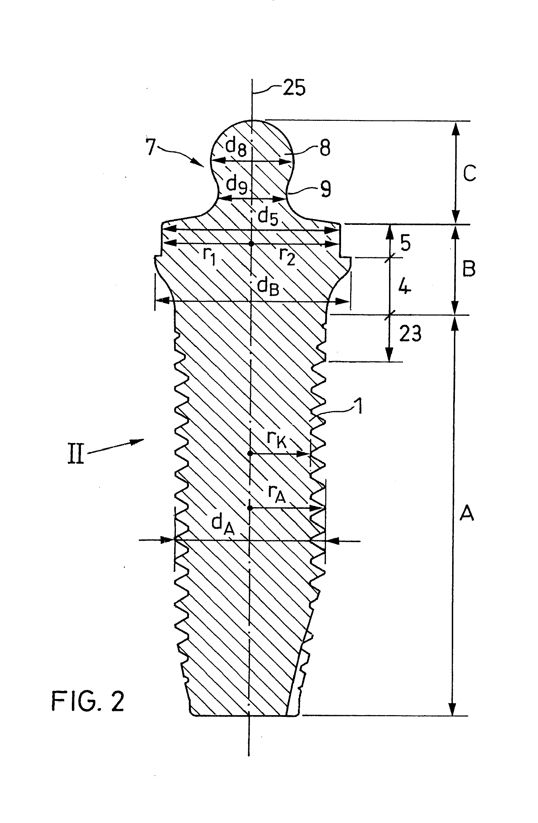

**Fig. 1a** **und** **1b** zeigen eine schematische Aufsicht auf die beispielhaften Ausführungsformen Ia und Ib des Keramikimplantates und Fig. 2 zeigt einen schematischen Längsschnitt durch die beispielhafte Ausführungsform II des Keramikimplantates mit einer axialen Implantatlänge L, einem distalen enossalen Gewindebereich A, einem gingvialen Zwischenbereich B, einem proximalen Bereich C und einer Implantatachse 25.

Der enossale Gewindebereich A der beispielhaften Keramikimplantate Ia, Ib und II ist mit einem Gewinde 1 ausgerüstet, welches optional mindestens eine Nut 2 und ebenfalls optional einen Gewindeauslauf 23 aufweist. Die Nut 2 öffnet sich zur distalen Implantatstirnseite. Der Zwischenbereich B weist in dieser beispielhaften Ausführungsform eine Erweiterungszone 4 und Eindrehgeometrie 5 auf.

Der proximale Bereich C ist mit mindestens einer Struktur zur Befestigung eines ein- oder mehrteiligen Aufbauelementes ausgerüstet. Die beispielhaften Keramikimplantate Ia, Ib und II unterscheiden sich nur im proximalen Bereich C.

In der beispielhaften Ausführungsform Ia dargestellt in Fig. 1a ist der proximale Bereich C als ein konischer Pfosten 6a zur Aufnahme eines Aufbauelementes ausgebildet. Die Mittelachse des Pfostens 6a der Ausführungsform Ia stimmt mit der Implantatachse 25 in dem Zwischenbereich B und dem enossalen Gewindebereich A überein.

In der beispielhaften Ausführungsform Ib dargestellt in Fig. 1b ist der proximale Bereich C als ein abgewinkelter Pfosten 6b ausgebildet. In manchen Ausführungsformen ist der Pfosten maximal so stark abgewinkelt, dass der Pfosten nicht über den Innenradius r₂ der nicht rotationssymmetrischen Aussenstruktur der Eindrehgeometrie 5 hinausragt. Dies ist in der Fig. 1b durch die Hilfslinie h dargestellt. Vorteilhafterweise ist bei Ausführungsformen mit einem abgewinkelten Pfosten, welcher nicht über den Innenradius der nicht rotationssymmetrischen Aussenstruktur der Eindrehgeometrie 5 hinausragt, diese Eindrehgeometrie für das gleiche Eindrehwerkzeug von proximaler Seite her zugänglich wie für eine entsprechende Ausführungsform des Keramikimplantates mit beispielsweise einem konischen Pfosten, dessen Mittelachse mit der Implantatachse 25 im Zwischenbereich B und im enossalen Gewindbereich A übereinstimmt.

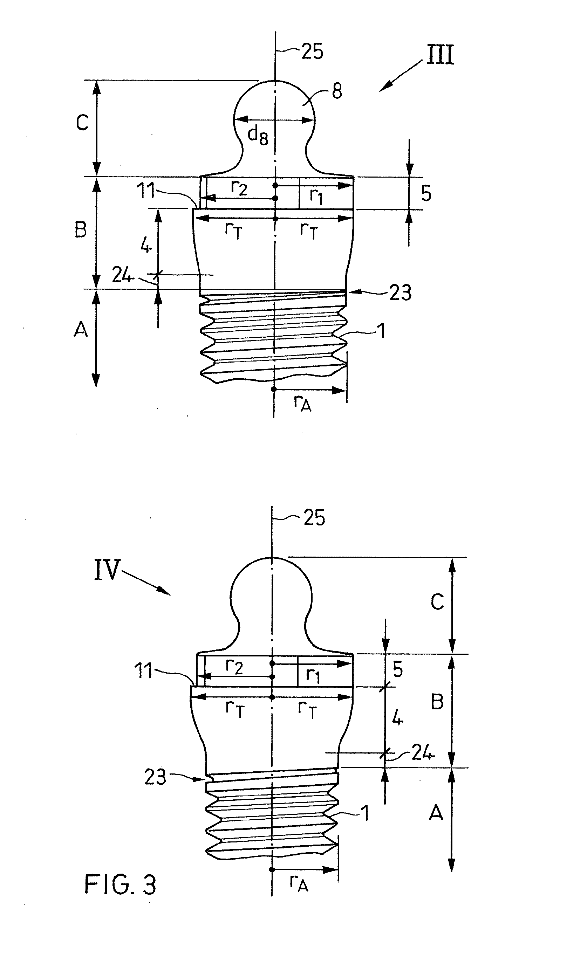

In der beispielhaften Ausführungsform II dargestellt in Fig. 2 und Fig. 3 ist der proximale Bereich als Kugelanker 7 zur Aufnahme eines Aufbauelementes ausgebildet und weist ein Köpfchen 8 und eine Einschnürung 9 auf.

Der Gewindebereich A der beispielhaften Keramikimplantate Ia, Ib und II weist ein selbstschneidendes Gewinde 1 auf mit Schneidekanten 3 des Gewindes 1 an der Nut 2. Die Nut 2 kann gegebenenfalls beim Eindrehen abgeschnittenes Knochenmaterial aufnehmen. Während die dargestellte beispielhafte Nut 2 axial verläuft, kann sie in andern Ausführungsformen beispielsweise helikal statt axial verlaufen.

In **Fig. 2** sind Implantatdurchmesser d_{A}, d_{B}, d₅, d₈ und d₉ eingezeichnet. Dabei bezeichnen d_{A} und d_{B} beispielhafte Implantatdurchmesser im Gewindebereich A und im Zwischenbereich B des Keramikimplantates. d₅ bezeichnet einen beispielhaften Implantatdurchmesser der Eindrehgeometrie 5 im Bereich B. Im Zwischenbereich B bezeichnet r₁ einen Aussenradius und r₂ einen Innenradius der nicht rotationssymmetrischen Aussenstruktur der Eindrehgeometrie 5. Im proximalen Bereich C bezeichnet d₈ den Durchmesser eines runden Köpfchens 8 und d₉ eine Taille, also den kleinsten Durchmesser einer Einschnürung 9 des proximalen Bereichs C.

Fig. 2 zeigt, dass jeder Implantatdurchmesser im Zwischenbereich B wie der beispielhaft eingezeichnete Implantatdurchmesser d_{B} mindestens gleichgross beziehungsweise grösser ist der doppelte Kernradius r_{K} des Gewindes 1 im Gewindebereich A. Da die Eindrehgeometrie 5 im Zwischenbereich B angeordnet ist, muss auch jeder Implantatdurchmesser d₅ der Eindrehgeometrie 5 grösser sein als, beziehungsweise gleich gross sein wie der doppelte Kernradius des Gewindes r_{K} im Gewindebereich A. In manchen Ausführungsformen ist auch der doppelte Innenradius r₂ der nicht rotationssymmetrischen Aussenstruktur der Eindrehgeometrie 5 mindestens gleich gross wie der doppelte Kernradius des Gewindes r_{K}. In den beispielhaften Ausführungsformen dargestellt in Fig. 1 und in Fig. 2 sind Kernradius r_{K} und der Aussenradius r_{A} über den grössten Teil der axialen Länge des Gewindes 1 konstant und gegen die distale Stirnseite des Keramikimplantates hin nehmen der Kernradius r_{K} und der Aussenradius r_{A} in distaler Richtung ab während am proximalen Ende des Gewindebereichs A ein Gewindeauslauf 23 angeordnet ist, in welchem der Kerndurchmesser r_{K} in proximaler Richtung zunimmt während der Aussenradius r_{A} unverändert bleibt..

Die beispielhaften Ausführungsformen I und II des Keramikimplantates weisen eine Ausführungsform mit einer tulpenförmige Erweiterungszone 4 auf. Der Implantatdurchmesser erweitert sich beginnend am distalen Ende der Erweiterungszone an der Grenze zum Gewindebereich A in proximaler Richtung in einer beispielhaft zuerst konvexen und dann konkaven S-Kurve. In proximaler Richtung an die Erweiterungszone 4 angrenzend ist die Eindrehgeometrie 5 angeordnet, hier beispielhaft mit einem etwas verkleinerten Implantatdurchmesser. Dadurch wird am proximalen Ende der Erweiterungszone 4, beziehungsweise am distalen Ende der Eindrehgeometrie 5, eine Schulter 11 ausgebildet. Diese Schulter 11 hat eine vorteilhafte Stoppwirkung auf ein Eindrehwerkzeug, das von proximaler Seite her an die Eindrehgeometrie 5 angesetzt wird.

Die axiale Gesamtlänge L von manchen Ausführungsformen des Keramikimplantates liegt beispielsweise in einem Bereich von 10 bis 25 mm und insbesondere in einem Bereich mit einer Untergrenze eines Wertes zwischen 11 bis 18 mm und einer Obergrenze eines Wertes zwischen 19 bis 24 mm.

Die nachfolgend genannten Längen beziehen sich jeweils auf die axiale Länge eines Implantatbereiches von manchen Ausführungsformen des Keramikimplantates, wobei spezifische Längen eines Implantatbereiches frei mit spezifischen Längen eines andern Implantatbereiches des Keramikimplantates kombinierbar sind. Der distale enossale Gewindebereich A zur Verankerung des Keramikimplantats im Knochen weist beispielsweise eine Länge auf in einem Bereich von 8 bis 16 mm und insbesondere in einem Bereich mit einer Untergrenze von 9, 10, 11 oder 12 mm und einer Obergrenze von 11, 12, 13, 14 oder 15 mm. Der Zwischenbereich B weist beispielsweise eine Länge auf in einem Bereich von 1 bis 4 mm und insbesondere in einem Bereich mit einer Untergrenze von 1, 1.5 oder 2 mm und einer Obergrenze von 2.5, 3 oder 3.5 mm. Der proximale Bereich A mit mindestens einer Struktur zur Befestigung eines ein- oder mehrteiligen Aufbauelementes weist beispielsweise eine Länge auf in einem Bereich von 1.5 bis 6 mm und insbesondere in einem Bereich mit einer Untergrenze von 1.5, 2.0, 2.5, oder 3.0 mm und einer Obergrenze von 2.5, 2.75, 2.9, 3.0, 3.1, 3.25, 3.5, 4, 4.5 oder 5 mm auf.

Manche beispielhafte Ausführungsformen eines Keramikimplantatsystems enthalten beispielsweise Keramikimplantate mit einer axialen Länge des distalen enossalen Bereiches A von 8 bis 16 mm oder 9 bis 14 mm oder 10.5 bis 12 mm, insbesondere 11.25 mm, einer axialen Länge des Zwischenbereichs B von 2 bis 3 mm, insbesondere 2.5 mm und einer axialen Länge des proximalen Bereichs C von 2 bis 3.5 mm, insbesondere 2.5 bis 3 mm oder 2,7 bis 2.8 mm auf.

Die axiale Länge der Eindrehgeometrie 5 ist in manchen Ausführungsformen im Bereich von 0.5 bis 1.5 mm, insbesondere von 0.8 bis 1.2 mm oder 0.9 mm bis 1.1 mm.

Manche Ausführungsformen des Keramikimplantats mit einer Lockball Patrize weisen eine axiale Länge des Gewindebereichs A von einem Wert zwischen beispielsweise 7 mm und 13 mm auf, insbesondere von 8.5 mm, 10 mm und 11.5 mm auf, des Zwischenbereichs B für Gingviahöhen von beispielsweise 2 mm bis 5 mm, insbesondere 2.0 mm, 2.5 mm, 3.0 mm, 3.5 mm, 4.0 mm oder 4.5 mm und des proximalen Bereichs von beispielsweise 2.9 mm bis 3.9 mm, insbesondere 3.4 mm bei einem doppelten Kernradius r_{K} des Gewindes von beispielsweise 3.9 bis 5 mm, insbesondere 4.1 bis 4.7 mm. Im Zwischenbereich weisen manche Ausführungsformen eine axiale Länge der Eindrehgeometrie von 0.4 bis 1.4 mm, insbesondere 0.7 bis 1.1 mm und der Erweiterungszone von 1.5 bis 2.5 mm insbesondere 1.8 bis 2.2 mm auf oder eine andere Kombination, welche die gewünschte axiale Länge des Zwischenbereichs für eine entsprechende Gingviahöhe ergibt.

Manche Ausführungsformen des Keramikimplantatsystems enthalten alternativ oder zusätzlich zu den oben beschriebenen unterschiedlichen Ausführungsformen des Keramikimplantats, Ausführungsformen bei welchen sich der Gewindedurchmesser bzw. der Implantatdurchmesser am proximalen Ende des Gewindebereichs unterscheidet. Am proximalen Ende des Gewindebereichs misst der Implantatdurchmesser beispielsweise zwischen 3 und 4.5 mm oder insbesondere zwischen 3.5 mm und 4 mm.

**Fig. 3** zeigt beispielhafte Ausführungsformen III und IV des Keramikimplantats in schematischen Teilaufsichten mit dem proximalen Teil C und dem Zwischenbereich B aufweisend eine Eindrehgeometrie 5, eine optionale Erweiterungszone 4 und eine optionale Abstandszone 24 sowie mit einem nur unvollständig abgebildeten Gewindebereich A, von welchem ein distaler Teil des Gewindes und das distale Ende des Gewindebereichs A nicht gezeichnet sind.

Der proximale Teil C der Ausführungsformen III und IV ist identisch ausgestaltet und weist einen Kugelanker, beispielsweise eine Lockball Patrize für eine handelsübliche Matrize auf.

Auch die an das distale Ende des proximalen Teils angrenzende Eindrehgeometrie (5) beider Ausführungsformen III und IV weist eine gleiche nicht rotationssymmetrische Aussenstruktur auf, hier beispielhaft ein Gleichdick mit gleichen Aussenradien r₁ und gleichen Innenradien r₂.

Hingegen weisen die beiden beispielhaften Ausführungsformen des Keramikimplantats III und IV einen unterschiedlichem doppelten Aussenradius r_{A} des Gewindes 1 auf und somit einen unterschiedlichen Implantatdurchmesser am proximalen Ende des Gewindes auf. Dieser Unterschied beträgt beispielsweise weniger als 1 mm, insbesondere 0.1 mm bis 0.8 mm oder 0.2 bis 0.6 mm oder 0.3 bis 0.5 beispielsweise 0.4 mm.

Die Aussenradien r₁ der Eindrehgeometrien 5 der beispielhaft dargestellten Ausführungsformen III und IV sind gleich gross wie der Radius r_{T} der Erweiterungszone 4 an ihrem proximalen Ende. Die Innenradien r₂ der nicht rotationssymmetrischen Aussenstruktur der Eindrehgeometrien 5 der beispielhaft dargestellten Ausführungsformen III und IV sind beispielsweise 0.025 mm bis 0.3 mm kleiner als die Aussenradien r₁ insbesondere 0.05 mm bis 0.15 mm oder 0.07 bis 0.1 mm kleiner als die Aussenradien r₁ bzw. als der Radius r_{T} am proximalen Ende der Erweiterungszone 4. Der Aussenradius r₁ der Aussenstruktur der Eindrehgeometrie misst beispielsweise 1.5 mm bis 2.5 mm, insbesondere 1.8 mm bis 2.2 mm. Ein Durchmesser d₅ der Eindrehgeometrie misst beispielsweise 3.5 bis 5 mm, insbesondere 3.8 mm bis 4.7 mm oder 4 mm bis 4.5 mm.

Die beiden in Fig. 3 dargestellten Ausführungsbeispiele des Keramikimplantates sind in einem beispielhaften Keramikimplantatsystem enthalten, bei welchen die unterschiedliche Ausgestaltung der Erweiterungszonen 4 so auf die unterschiedlichen Gewindedurchmesser abgestimmt ist, dass die Erweiterungszone beider Ausführungsformen III und IV an ihrem distalen Ende einen Implantatdurchmesser aufweist, der gleich gross ist wie der jeweilige Implantatdurchmesser am proximalen Ende des Gewindebereichs bzw. wie der doppelte Aussenradius r_{A} des Gewindes und dass die Erweiterungszone an ihrem proximalen Ende einen Implantatdurchmesser aufweist, der gleich gross ist wie der gleiche doppelte Aussenradius r₁ der gleichen Aussenstruktur der Eindrehgeometrie 5.

Die beispielhaften Ausführungsformen III und IV des beispielhaften Keramikimplantatsystems weisen zusätzlich zur Erweiterungszone 4 im Zwischenbereich B eine optionale Abstandszone 24 auf, welche zwischen dem proximalen Ende des Gewindebereichs A und dem distalen Ende der Erweiterungszone 4 angeordnet ist. In der Abstandszone erweitert sich der Implantatdurchmesser im Vergleich zum Implantatsdurchmesser am proximalen Ende des Gewindebereichs nicht und er ist insbesondere wie in den Ausführungsformen III und IV gleich gross wie der doppelte Aussenradius r_{A} des Gewindes.

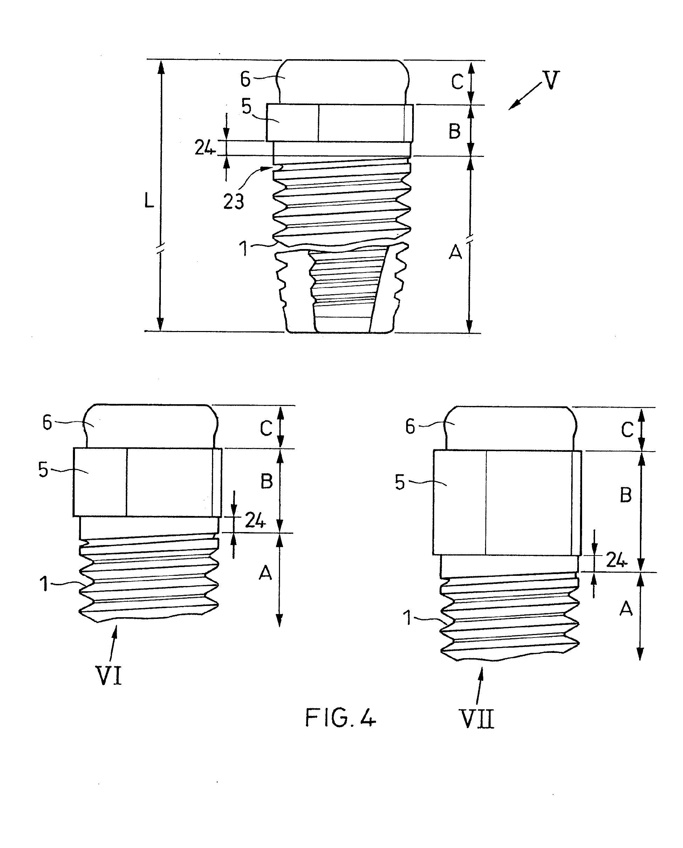

**Fig. 4** zeigt schematische Teilaufsichten auf drei weitere beispielhafte Keramikimplantate V, welche im proximalen Bereich eine Locator Patrize aufweisen. Solche und optional weitere Ausführungsbeispiele des Keramikimplantats sind beispielsweise in einem Keramikimplantatsystem enthalten.

Manche Ausführungsformen des Keramikimplantats mit einer Locator Patrize weisen eine axiale Länge des Gewindebereichs A von beispielsweise 8 bis 12 mm, insbesondere von 8.5 mm, 9.0 mm, 9.5 mm oder 10.0 mm bis 10.5 mm, 11.0 mm oder 11.5 mm auf, eine axiale Länge des Zwischenbereichs B für verschiedene Gingviahöhen von beispielsweise 2 mm bis 5 mm, insbesondere von 2.5 mm, 3.0 mm oder 3.5 mm bis 3.5 mm, 4.0 mm oder 4.5 mm und des proximalen Bereichs von beispielsweise 2.5 bis 4.5, insbesondere 3.0 bis 4.0 mm auf. Der Implantatdurchmesser dieser Ausführungsformen im proximalen Gewindebereich misst beispielsweise zwischen 3 mm und 4.5 mm, insbesondere von 3.5 mm bis 4.0 mm.

Der Zwischenbereich B ist in manchen Ausführungsformen wie beispielsweise der Keramikimplantate V, VI und VII in Fig. 4 ohne Erweiterungszone und ohne Abstandszone über seine ganze axiale Länge als nicht rotationssymmetrische Eindrehgeometrie ausgestaltet. Die axialen Längen der Eindrehgeometrien 5 betragen zwischen 2 und 5 mm, jene der gezeichneten der Implantate V, VI und VII messen beispielsweise 2.5 mm, 3.5 mm und 4.5 mm. In andern nicht gezeichneten Ausführungsformen ist der Zwischenbereich nicht über die ganze axiale Länge als Eindrehgeometrie ausgestaltet und weist zusätzlich eine Abstandszone und oder eine Erweiterungszone auf.

In den beispielhaft dargestellten Ausführungsformen V, VI und VII des Keramikimplantats ist der Aussenradius der Eindrehgeometrie 5 grösser als der Ausserradius des Gewindes.

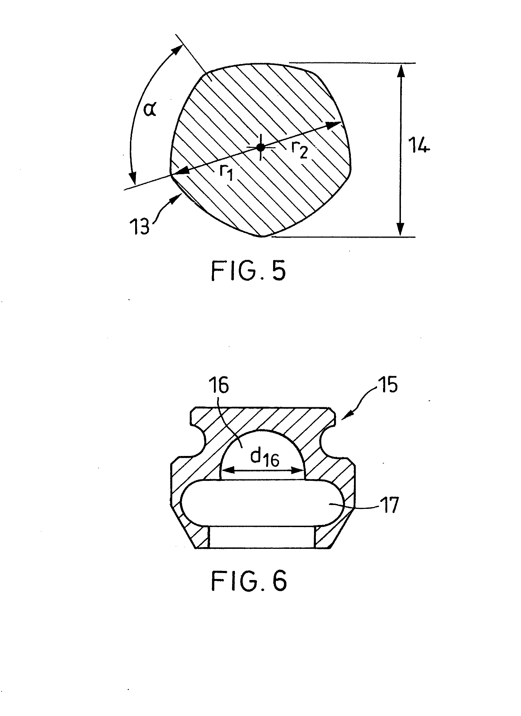

**Fig. 5** zeigt einen Querschnitt durch die Eindrehgeometrie 5 der in den Figuren 1 bis 4 beispielhaften Ausführungsformen des Keramikimplantates Diese Eindrehgeometrie 5 ist als nicht rotationssymmetrische Aussenstruktur beispielhaft in der Form eines Gleichdick 13 ausgebildet.

Selbstverständlich ist die in Fig. 5 dargestellte Ausführungsform der Eindrehgeometrie 5 für andere Ausführungsformen des Keramikimplantates als den oben genannten Ausführungsbeispielen verwendbar und ebenso selbstverständlich könnten die dargestellten Ausführungsbeispiele des Keramikimplantates auch mit einer anderen nicht rotationsymmetrischen Aussenstruktur als Eindrehgeometrie ausgerüstet werden, beispielsweise mit einem anderen Gleichdick oder mit einem Aussenkant usw.

Die in Fig. 5 beispielhaft dargestellte nicht rotationssymmetrische Aussenstruktur der Eindrehgeometrie 5 ist ein Gleichdick 13 , welches ein regelmässiges Gleichdick ist, basierend auf einem regelmässigen Fünfeck mit einem Winkel α von 72°, mit einer definitionsgemäss konstanten Breite 14. Das Mass der konstanten Breite 14 liegt beispielsweise in einem Bereich mit einer Untergrenze von 3, 3.25, 3.5, 3.75 4, 4.25, 4.5, 4.75 oder 5.0 mm und einer Obergrenze in einem Bereich von 4.5, 4.75, 5.0, 5.5, 5.75 oder 6 mm und liegt insbesondere in einem Bereich von 3.5 bis 5.5 mm.

In andern nicht gezeichneten Ausführungsformen der Eindrehgeometrie 5 im Zwischenbereich B ist die nicht rotationssymmetrische Aussenstruktur beispielsweise ein unregelmässiges Gleichdick oder ein Aussenkant. In manchen Ausführungsformen mit einem Aussenkant misst der Implantatdurchmesser der Eindrehgeometrie beispielsweise 3.5 bis 6 mm, insbesondere 4 bis 5mm.

Figuren 1a, 1b, 2 und 3 zeigen, dass die Eindrehgeometrie 5 in den beispielhaften Ausführungsformen Ia, Ib und II bis IV im Vergleich zur distal angrenzenden Erweiterungszone 4 zurückversetzt ist, das heisst, dass der Aussenradius r₁ der nicht rotationssymmetrischen Eindrehgeometrie ist kleiner oder insbesondere gleich gross wie der Aussenradius r_{T} der distal angrenzenden Erweiterungszone 4, und der Innenradius r₂ der Eindrehgeometrie 5 ist kleiner als der Aussenradius r_{T} der distal angrenzenden Erweiterungszone 4 ist. In diesen und manchen weiteren Ausführungsformen ist also der Implantatdurchmesser d₅ der zurückversetzten Eindrehgeometrie 5 kleiner als der Implantatdurchmesser des distal an die Eindrehgeometrie 5 angrenzenden Implantatbereichs, wie beispielsweise einer distal angrenzenden Erweiterungszone 4, einer distal angrenzenden Abstandszone 24 oder eines distal angrenzenden Gewindebereichs A.

In andern Ausführungsformen des Keramikimplantates steht die Eindrehgeometrie 5 gegenüber einem distal an die Eindrehgeometrie 5 angrenzenden Implantatbereich hervor, wie beispielsweise gegenüber einer distal angrenzenden Erweiterungszone 4, einer distal angrenzenden Abstandszone 24 oder eines distal angrenzenden Gewindebereichs A. Insbesondere ist in solchen Ausführungsformen des Keramikimplantates der Implantatdurchmesser d₅ der Eindrehgeometrie 5 grösser und insbesondere der Aussenradius r₁ der Aussenstruktur der Eindrehgeometrie 5 grösser als der Durchmesser oder der grösste Radius in einer Schnittfläche senkrecht zur Achse durch das Keramikimplantat unmittelbar distal zur Eindrehgeometrie am proximalen Ende beispielsweise der optionalen Erweiterungszone 4 oder der optionalen Abstandszone 24 oder des Gewindebereichs A. Die Ausführungsformen V, VI und VII des Keramikimplantates dargestellt in Fig. 4 weisen eine solche gegenüber der distal angrenzenden Abstandszone hervorstehende Eindrehgeometrie 5 auf.

**Fig. 6** zeigt einen Querschnitte durch eine beispielhafte Ausführungsform eines Teils 15 eines mehrteiligen Aufbauelementes eines Zahnimplantatsystems. Der Teil 15 ist ein Halteelement, welches im proximalen Bereich C des Keramikimplantates befestigbar ist und welches mit mindestens einem weiteren Teil des Aufbauelementes verbindbar ist und z.B. einen Zahnersatz wie eine Brücke, Prothese oder Zahnkrone hält.

Das in Fig. 6 beispielhaft dargestellte Halteelement 15 ist als Matrize ausgestaltet und weist einen halbkugelförmigen Hohlraum 16 zur Aufnahme des Köpfchens 8 des Kugelankers 7 auf, wobei der Durchmesser d₁₆ des Hohlraums 16 an den Köpfchendurchmesser d₈ angepasst ist.

In der beispielhaften Ausführungsform des Aufbauelementes gemäss Fig. 6 weist das als Matrize ausgestaltete Halteelement 15 eine Vertiefung 17 auf für ein Schnappelement 18, beispielsweise für einen Schnappring, welcher insbesondere im Bereich der Einschnürung 9 des Keramikimplantates zwischen die Oberfläche des Keramikimplantates und die Innenwand der Matrize in die Vertiefung 17 eingefügt wird.

## Patentansprüche

1. Einstückiges keramisches Zahnimplantat, aufweisend
einen distalen enossalen Gewindebereich (A), in welchem ein Gewinde (1) angeordnet ist mit einem Kernradius (r_{K}) und einem Aussenradius (r_{A}) des Gewindes (1),
einen Zwischenbereich (B), in welchem eine proximal zugängliche Eindrehgeometrie (5) zum Eindrehen des Gewindebereichs (A) in ein Knochengewebe angeordnet ist, wobei die Eindrehgeometrie (5) als nicht rotationssymmetrische Aussenstruktur ausgebildet ist,
und einen proximalen Bereich (C) mit mindestens einer Struktur zur Befestigung eines ein- oder mehrteiligen Aufbauelementes,
**dadurch gekennzeichnet, dass** ein Implantatdurchmesser in jeder Schnittfläche senkrecht zur Implantatachse (25) im Zwischenbereich (B) und somit auch durch die im Zwischenbereich (B) angeordnete Eindrehgeometrie (5) mindestens gleich gross ist, wie der doppelte Kernradius (r_{K}) des Gewindes (1).

2. Keramikimplantat nach Anspruch 1, mit einer nicht rotationssymmetrischen Aussenstruktur der Eindrehgeometrie aufweisend einen Aussenradius (r₁) und einen Innenradius (r₂), wobei der Innenradius (r₂) der Eindrehgeometrie (5) mindestens gleich gross ist wie der Kernradius (r_{K}) des Gewindes.

3. Keramikimplantat nach Anspruch 2, wobei der Unterschied zwischen dem Aussenradius (r₁) und dem Innenradius (r₂) der nicht rotationssymmetrischen Aussenstruktur der Eindrehgeometrie (5) kleiner als 0.3 mm oder 0.2 mm ist, und insbesondere zwischen 0.13 mm und 0.18 mm misst.

4. Keramikimplantat nach Anspruch 2 oder 3, wobei der Zwischenbereich zusätzlich proximal zum Gewindebereich und distal zur Eindrehgeometrie eine insbesondere tulpenförmige Erweiterungszone aufweist, wobei der Implantatdurchmesser am distalen Ende der Erweiterungszone mindestens gleich gross ist wie der doppelte Aussenradius (r_{A}) des Gewindes und am proximalen Ende der Erweiterungszone grösser ist als an ihrem distalen Ende.

5. Keramikimplantat nach einem der Ansprüche 2 bis 4, wobei der Aussenradius (r₁) der Eindrehgeometrie (5) mindestens gleich gross ist wie der Aussenradius (r_{A}) des Gewindes.

6. Keramikimplantat nach Anspruch 4, wobei der Aussenradius (r₁) der Eindrehgeometrie (5) kleiner oder gleich gross ist wie ein Ausserradius (r_{T}) der insbesondere tulpenförmigen Erweiterungszone an deren proximalen Ende.

7. Keramikimplantat nach einem der Ansprüche 1 bis 6, wobei die Eindrehgeometrie (5) ein Aussen-Sechs-, Aussen-Acht- oder Aussen-Zwöltkant ist oder eine konvexe oder konkave mehrlappige Struktur, insbesondere ein konkaves und/oder insbesondere ein regelmässiges oder unregelmässiges Gleichdick ist.

8. Keramikimplantat nach einem der Ansprüche 1 bis 7, wobei die Eindrehgeometrie (5) zylindrisch oder konisch ist.

9. Keramikimplantat nach einem der Ansprüche 2 bis 8, wobei der Implantatdurchmesser in jeder Schnittfläche senkrecht zur Implantatachse im proximalen Bereich (C) gleich gross oder kleiner ist als der doppelte Innenradius (r₂) der Eindrehgeometrie (5).

10. Keramikimplantat nach einem der Ansprüche 1 bis 9, wobei die mindestens eine Struktur im proximalen Bereich (C) zur Befestigung des ein- oder mehrteiligen Aufbauelementes als Patrize (7) zur Verbindung mit einer Matrize ausgebildet ist oder eine Patrize zur Verbindung mit einer Matrize enthält oder wobei er proximale Bereich C als Pfosten 6a oder als abgewinkelter Pfosten 6b ausgestaltet ist.

11. Keramikimplantatsystem aufweisend mindestens zwei Keramikimplantate nach einem der Ansprüche 1 bis 10 mit einer gleichen nicht rotationssymmetrischen Aussengeometrie der Eindrehgeometrie (5), wobei die Keramikimplantate des Keramikimplantatsystems sich mindestens durch die axiale Länge der Eindrehgeometrie (5) und / oder in der Ausgestaltung mindestens eines anderen Implantatsbereichs, insbesondere des enossalen Gewindebereichs (C), des proximalen Bereich (A), der optionalen Abstandszone (24) oder der optionalen Erweiterungszone (4) unterscheiden.

12. Zahnimplantatsystem aufweisend mindestens ein Keramikimplantat nach Anspruch 1 bis 10 oder ein Keramikimplantatsystem nach Anspruch 11 und zusätzlich aufweisend mindestens ein einteiliges Aufbauelement oder mindestens einen Teil (15) eines mehrteiligen Aufbauelementes, insbesondere ein Halteelement.

13. Zahnimplantatsystem nach Anspruch 12, wobei das Aufbauelement oder mindestens ein Teil (15) des Aufbauelementes mit dem Keramikimplantat über eine Schnappverbindung oder eine Presspassung verbindbar ist.

14. Zahnimplantatsystem nach Anspruch 11 oder 12 zusätzlich aufweisend ein Schnappelement (18), insbesondere einen Schnappring, wobei insbesondere das Schnappelement Silikon enthält und insbesondere ein Silikonring ist.

15. Set enthaltend ein Keramikimplantat nach einem der Ansprüche 1 bis 10 oder ein Keramikimplantatsystem nach Anspruch 11 oder ein Zahnimplantatsystem nach einem der Ansprüche 12 bis 14 zusätzlich enthaltend ein Eindrehwerkzeug für das Keramikimplantat.
